(19) Europäisches Patentamt
European Patent Office
Office européen des brevets

(11) Numéro de publication: **0 294 307 B1**

(12) **FASCICULE DE BREVET EUROPEEN**

(45) Date de publication de fascicule du brevet: **22.06.94**

(51) Int. Cl.$^5$: **C07D 263/26**, C07D 263/28, C07D 413/12, A61K 31/495

(21) Numéro de dépôt: **88450019.0**

(22) Date de dépôt: **09.05.88**

Le dossier contient des informations techniques présentées postérieurement au dépôt de la demande et ne figurant pas dans le présent fascicule.

(54) **Arylcarbamoyl-3 aminométhyl-5 oxazolidines ainsi que leurs sels avec des acides pharmacologiquement compatibles.**

(30) Priorité: **14.05.87 FR 8706890**
**05.05.88 FR 8806253**

(43) Date de publication de la demande:
**07.12.88 Bulletin 88/49**

(45) Mention de la délivrance du brevet:
**22.06.94 Bulletin 94/25**

(84) Etats contractants désignés:
**AT BE CH DE ES GB IT LI LU NL**

(56) Documents cités:
**EP-A- 0 105 821**
**FR-A- 2 226 165**
**GB-A- 2 059 961**

**CHEMICAL ABSTRACTS, vol. 91, no. 25, 17 décembre 1979, page 676, résumé no.211309z, Columbus, Ohio, US; Y. YAMAMOTO et al.: "Studies on thiazolinederivatives. XV. Reactions of 2-phenyl and 2-methylamino-2-thiazolines withmethyl and phenylisothiocyanates", & KYORITSU YAKKA DAIGAKU KENKYU NEMPO 1978,(23), 38-46**

(73) Titulaire: **CORTIAL S.A.**
**7 rue de l'Armorique**
**F-75015 Paris(FR)**

(72) Inventeur: **Jarry, Christian**
**11 rue des Mésanges**
**F-33370 Artigues(FR)**
Inventeur: **Bosc, Jean-Jacques**
**16 Les Prés de l'Eglise**
**Pompignac**
**F-33370 Tresse(FR)**
Inventeur: **Panconi, Emmanuel**
**Rue du Maréchal Galliéni**
**F-33700 Merignac(FR)**
Inventeur: **Vaugien, Bernard**
**Résidence les Tourelles de la Valère**
**Avenue Aristide Briand F-33700**
**Merignac(FR)**

(74) Mandataire: **Phélip, Bruno et al**
**c/o Cabinet Harlé & Phélip**
**21, rue de La Rochefoucauld**
**F-75009 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 294 307 B1

## Description

La présente invention concerne de nouvelles arylcarbamoyl-3 aminométhyl-5 oxazolidines ainsi que leurs sels avec des acides pharmaceutiquement compatibles .

Les composés de l'invention ont pour formule générale :

(I) 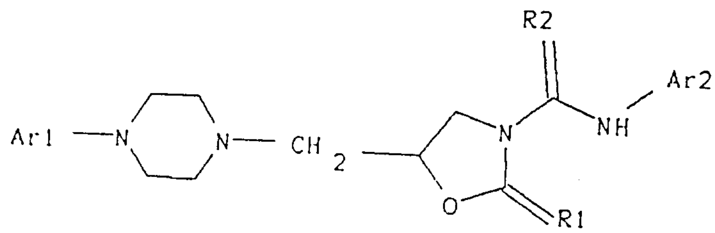

dans laquelle R1 est un oxygène ou un groupe NH, $R_2$ est un oxygène ou un soufre, $Ar_1$ est un groupe phényl substitué ou non par un ou plusieurs groupes choisis parmi des groupes alkyl de C1 à C4, halogène , chlore, brome, fluor, un groupe trifluorométhyl ou un groupe méthoxy, le groupe $Ar_1$ peut être également un groupe pyridyl ou pyrimidyl; $Ar_2$ est un groupe phényl substitué ou non par un ou plusieurs groupes choisis parmi des groupes alkyl de C1 à C4, chlore, brome, fluor, iode.

Les composés de l'invention sont préparés à partir des composés que la demanderesse a décrit dans FR-A-83 08 477.

(II) 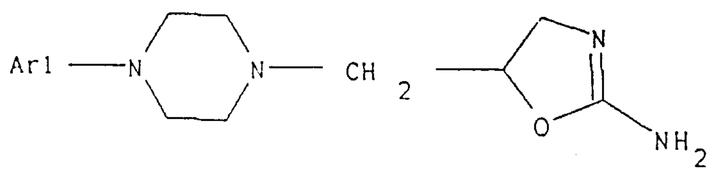

dans laquelle $Ar_1$ prend les mêmes valeurs que définies ci-dessus, par condensation, en milieu anhydre, à une température comprise entre 233°K et 293°K de préférence choisie entre 263°K et 293°K avec un isocyanate de phényle ou un isothiocyanate de phényle

III $AR_2\text{-}N=C=R_2$

dans lequel le groupe $R_2$ et le groupe $Ar_2$ prennent les valeurs définies précédemment.

Les produits de structure I avec $R_1 = O$ sont obtenus par hydrolyse acide et à l'ebulition du solvant des produits de structure I Avec $R_1 = NH$.

La demanderesse a déja décrit, dans le brevet FR-A- 83 13013, la reaction de l'isocyanate de phényle sur des composés de structure II et conduisant à des N-(aminométhyl-5 oxazolin-2-yl-2) N'-phényl urée de structure IV

IV 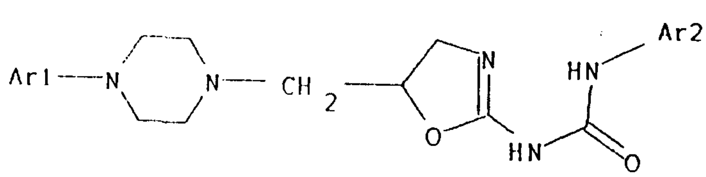

YAMAMOTO et coll ont déja décrit la réaction de isothiocyanate de phényle sur la phénylamino-2 thiazoline dans la référence suivante, Kyoritsu Yakka Daigaku Kenkyu Nempo 1978, 23, 38-46, Chemical Abstracts volume 91, abstact N°211309. La réaction conduit à la phénylthiocarbamoyl-3 phénylmino-2 thiazoline. SIAGLO-II et coll ont décril dans la référence suivante Pol. J. Pharmacol. Pharm. 1975, 27, 1, 57-60, Chemical abstracts volume 83 abstract N°79157, la réaction de l' isothiocyanate de phényle sur l'imino-2 sélénazolidinone-4 qui conduit à la phénylthiocarbamoyl-3 imino-2 selénazolidinone-4 alors que la

réaction de l'isocyanate de phenyle conduit à la N-sélénazoline-2 one-4 phénylurée.

GB-A-2059961 divulgue des composés de structure suivante :

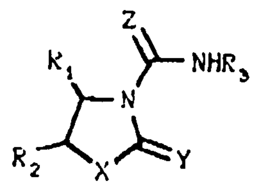

où X, Y et Z sont des atomes d'oxygène ou de soufre , $R_1$ est un groupe alkyle en $C_1$ en $C_4$, $R_2$ est un hétérocycle à 5 chaînons contenant des atomes d'oxygène ou de soufre, un groupe phényle substitué ou non par un ou deux groupes alkyles , halogène , halogénoalkyle, alkoxy, nitro ou benzodioxy ; $R_3$ est un cycloalcane ou un cycloalcène de 5 à 7 chaînons, ou un hétérocycle contenant des atomes d'oxygène , de soufre ou d'azote, substitué ou non.

Les composés sont préparés par réaction d'un composé :

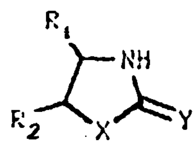

avec un isocyanate de formule $R_3NCZ$
où $R_1$, $R_2$, $R_3$, X, Y et Z ont la même significatlon que précédemment . Ces composés ont d'intéressantes propriétés acaricide .

La demande de certificat d'addition FR-A-2226165 décrit des composés de structure suivante:

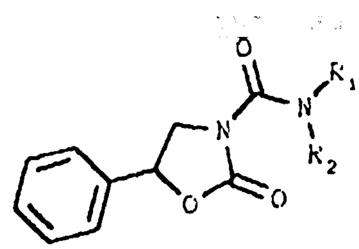

où N($R_1$, $R_2$) représente :

- un groupe dialkylamino où les termes alkyles comprennent de 1 à 4 atomes de carbone,
- un reste hétérocyclique choisi parmi les suivants : pyrrolidino, morpholino , pipéridino et hexaméthylèneimino.

Les composés sont préparés par condensation du sel de sodium de la phényl-5 oxazolidinone-2 de formule :

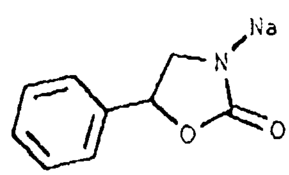

avec un chlorure de carbamyle de formule

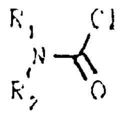

où $R_1$ et $R_2$ ont la même signification que précédemment .

Ces composés présentent d'intéressantes propriétés analgésiques et antiinflammatoires.

La demanderesse a maintenant trouvé que la réaction conduisant aux composés de structure (IV) procède en deux temps: a) obtention de l'arylcarbamoyl-3 imino-2 aminométhyl-5 oxazolidine qui est le produit cinétique de la réaction, b) obtention de la phénylurée de structure IV qui est le produit thermodynamique de la réaction.

La demanderesse a trouvé, après une étude cinétique de la réaction des composés de structure II avec l'isocyanate de phenyle, les conditions de température permettant d'atteindre les composés de l'invention. Cette étude a été réalisée de la façon suivante: dans dix tubes à essai de 20 $cm^3$ il a ete dissout 100 mg du composé de structure II dans 10 $cm^3$ de benzène anhydre, les tubes sont mis dans des bains thermostatés de 273 ° K à 353 ° K l'augmentation de la température de ces bains est faite de 8 en 8 degres kelvin, on ajoute alors l'isocyanate de phényle, l'avancement de la réaction est suivie par chromatographie en couche mince. Les quantités des deux composés I et IV sont déterminées par photodensitométrie. A 273 ° K le compose I est seul présent alors que dès 297 ° K le composé IV représente 35% des composés de la réaction.

L'invention va être décrite de façon plus précise dans les exemples suivants sans toutefois que ceux-çi ne limitent sa portée.

Les points de fusion sont pris au banc Kofler, les déplacements chimiques sont exprimés en ppm par rapport au TMS utilisé comme étalon interne.

EXEMPLE 1

Phényl carbamoyl-3, imino-2, (phényl-4 pipérazinylméthyl)-5 oxazolidine ou COR3262 produit de structure I avec $R_1$ = NH, $R_2$ = O, $Ar_1$ = phényl, $Ar_2$ = phényl.

Synthèse

Dans un réacteur muni d'un réfrigerant, d'une ampoule à brome, d'un agitateur et d'un thermomètre on introduit 26 grammes (0,1 mole) d'amino-2 (phényl-4 pipérazinylméthyl)-5 oxazoline ou COR3224 dont la préparation est décrite dans le brevet FR 83 08477 préalablement dissoute dans 150 $cm^3$ de benzène anhydre. Le mélange est refroidi à 273 ° K puis on ajoute goutte à goutte 11,9 grammes (0,1 mole) d'isocyanate de phényl en controlant la température celle çi ne doit pas dépasser 293 ° K. Le mélange est laissé sous agitation pendant 25 minutes. Le solide qui a précipité est recueilli par filtration et est recristallisé dans l'oxyde de diisopropyle. On recueille 19 grammes de COR3262, rendement 51%, masse moléculaire 379.

Caractéristiques physicochimiques du COR3262

Point de fusion 163 ° C
Spectre de RMN dans le $CDCl_3$
2,5-3,4 ppm, 10 protons, massif complexe, 5 groupes N-$CH_2$; 3,6-4,5 ppm, 2 protons, massif complexe, 1 groupe N-$CH_2$ du cycle de l'oxazolidine; 4,5-5,0 ppm, 1 proton, multiplet, 1 CHO du cycle de l'oxazolidine; 6,0 ppm, 1 proton échangeable avec $D_2O$, pic large, NH du groupe imino; 6,7-7,7 ppm, 10 protons, massif complexe, protons aromatiques; 11,6 ppm, 1 proton échangeable avec $D_2O$, pic large, NH du groupe carbamoyl.

EXEMPLE 2

Chlorhydrate de la phényl carbamoyl-3, (phényl-4 pipérazinylméthyl)-5 oxazolidinone-2 ou COR3267 produit de structure I avec $R_1$ = O, $R_2$ = O, $Ar_1$ = phényl, $Ar_2$ = phényl.

Synthèse

Dans un réacteur de 250 $cm^3$ muni d'un réfrigérant et d'un agitateur on introduit dans 50 $cm^3$ d'eau 3,79 g (0,01 mole) de phényl carbamoyl-3 imino-2 (phényl-4 pipérazinylméthyl)-5 oxazolidine ou COR3262, préparé dans l'exemple 1, puis on ajoute en une seule fois 20 $cm^3$ d'acide chlorhydrique normal (0,02) mole. Le mélange est porté au reflux . Dès le début du chauffage tout le solide se solubilise, 15 minutes plus tard un abondant précipité se forme, le chauffage est maintenu pendant une heure. Le solide est recueilli par filtration et est recristallisé dans l'éthanol absolu. On recueille 1,8 gr de COR3267, rendement 44%, masse moléculaire 416,5.

Caractéristiques physicochimiques du COR3267

Point de fusion 134 ° C
Spectre de RMN dans le DMSO $D_6$
2,8-4,5 ppm, 12 protons, massif complexe, 6 groupes N-$CH_2$; 5,2-5,8 ppm, 1 proton, multiplet, 1 CHO du cycle de l'oxazolidinone; 6,7-7,7 ppm, 10 protons, massif complexe, protons aromatiques; 9,8 ppm, 1 proton échangeable avec $D_2O$, pic large,NH du groupe carbamoyl; 12,2 ppm, 1 proton échangeable avec $D_2O$, dôme, $NH^+$.

EXEMPLE 3

Phényl thiocarbamoyl-3 imino-2 (phényl-4 pipérazinylméthyl)-5 oxazolidine ou COR3296 produit de structure I avec $R_1$ = NH, $R_2$ = S, $Ar_1$ = phényl, $Ar_2$ = phényl.

Synthèse

La méthode de synthèse est la même que celle de l'exemple 1 sauf en ce que l'on utilise l'isothiocyenate de phényle. Le produit final est purifié par recristallisation dans l'éthanol absolu, rendement 45%, masse moléculaire, 395.

Caractéristiques physicochimiques du COR3296

Point de fusion 140 ° C
Spectre de RMN dans le DMSO $D_6$
2,4-5,2 ppm, 13 protons, massif complexe, 6 groupes N-$CH_2$ et 1 CHO du cycle de l'oxazolidine; 6,6-7,9 ppm, 11 protons, massif complexe, protons aromatiques plus NH du groupe imino; 14,2 ppm, 1 proton échangeable avec $D_2O$, dôme, NH du groupe carbamoyl.

EXEMPLE 4

Chlorhydrate de la phényl thiocarbamoyl-3 (phényl-4 pipérazinylméthyl)-5 oxazolidinone-2 ou COR4430 produit de stucture I avec $R_1$ = O, $R_2$ = S, $Ar_1$ = phényl, $Ar_2$ = phényl.

Synthèse

On procéde de la même façon que dans l'exemple 2 en utilisant le COR3296 comme produit de départ, on obtient après recristallisation dans l'éthanol absolu le COR4430 avec un rendement de 45%, masse moléculaire 432,5.

Caractéristiques physicochimiques du COR4430

Point de fusion 200 ° C
Spectre de RMN dans le DMSO $D_6$
2,9-4,7 ppm, 12 protons, massif complexe, 6 groupes N-$CH_2$; 5,2-5,7 ppm, 1 proton, multiplet, 1 CHO du cycle de l'oxazolidinone; 6,6-7,7 ppm, 10 protons, massif complexe, protons aromatiques; 11,4 ppm, 1 proton échangeable avec $D_2O$, singulet, 1 NH du groupe carbamoyl; 12,1 ppm, 1 proton échangeable avec $D_2O$, dôme, 1 $NH^+$.

EXEMPLE 5

(Chloro-4 phényl) carbamoyl-3 imino-2 (phényl-4 pipérazinylméthyl)-5 oxazolidine ou COR4429 produit de structure I avec $R_1$ = NH, $R_2$ = O, $Ar_1$ = phényl, $Ar_2$ = chloro-4 phényl.

Synthèse

La méthode de synthèse est la même que celle de l'exemple 1 sauf en ce que l'on utilise l'isocyanate de parachlorophényle. Le produit final est purifié par lavage à l'éthanol absolu, rendement 78%, masse moléculaire 413,5.

Caractéristiques physicochimiques du COR4429

Point de fusion 191 °C
Spectre de RMN dans le $CDCl_3$
2,6-3,4 ppm, 10 protons, massif complexe, 5 groupes N-$CH_2$; 3,6-4,5 ppm, 2 protons, massif complexe, 1 groupe N-$CH_2$ du cycle de l'oxazolidine; 4,4-5,0 ppm, 1 proton, multiplet, 1 CHO du cycle de l'oxazolidine; 6,0 ppm, 1 proton échangeable avec $D_2O$, NH du groupe imino; 6,7-7,6 ppm, 9 protons, massif complexe, protons aromatiques; 11,6 ppm, 1 proton échangeable avec $D_2O$, pic large, NH du groupe carbamoyl.

EXEMPLE 6

Phényl carbamoyl-3 imino-2 ((pyridyl-2)-4 pipérazinylméthyl)-5 oxazolidine ou COR3263 produit de structure I avec $R_1$ = NH, $R_2$ = O, $Ar_1$ = pyridyl-2, $Ar_2$ = phenyl.

Synthèse

a) Préparation de l'amino-2 ((pyridyl-2)-4 pipérazinylméthyl)-5 oxazoline ou COR3239 produit de structure II avec $Ar_1$ = pyridyl-2.
Le COR3239 est synthétisé selon la méthode décrite dans l'exemple 1 du brevet FR 83 08477. A partir de la (pyridyl-2)-pipérazine on obtient le COR3239, avec un rendement de 22%, masse moléculaire 261.

Caractéristiques physicochimiques du COR3239

Point de fusion 160 °C
Spectre de RMN dans le DMSO $D_6$
2,2-3,9 ppm, 12 protons, massif complexe, 6 groupes N-$CH_2$; 4,3-4,9 ppm, 1 proton, multiplet, 1 CHO du cycle de l'oxazoline; 5,8 ppm, 2 protons échangeables avec $D_2O$, pic large, $NH_2$; 6,4-8,2 ppm, 4 protons; massif complexe, protons aromatiques.

b) Préparation de la phényl carbamoyl-3 imino-2 ((pyridyl-2)-4 pipérazinylméthyl)-5 oxazolidine ou COR3263
En utilisant le mode opératoire de l'exemple 1 et à partir de 26,1 grammes (0,1 mole) de COR3239 et 11,9 grammes (0,1 mole) d'isocyanate de phényl on obtient, après recristallisation dans l'oxyde d' isopropyle, 18,5 grammes de COR3263, rendement 48%, masse moléculaire 380.

Caractéristiques physicochimiques du COR3263

Point de fusion 158 °C
Spectre de RMN dans le $CDCl_3$
2,5-4,5 ppm, 12 protons, massif complexe, 6 groupes N-$CH_2$; 4,5-5,0 ppm, 1 proton, multiplet, 1 CHO du cycle de l'oxazolidine; 6,0 ppm, 1 proton échangeable avec $D_2O$, pic large, NH du groupe imino; 6,5-8,3 ppm, 9 protons, massif complexe, protons aromatiques; 11,6 ppm, 1 proton échangeable avec $D_2O$, pic large, NH du groupe carbamoyl.

EXEMPLE 7

Phényl carbamoyl-3 imino-2 ((chloto-3 phényl)-4 pipérazinylméthyl)-5 oxazolidine ou COR3279 produit de structure I avec $R_1$ = NH, $R_2$ = O, $Ar_1$ = chloro-3 phényl, $Ar_2$ = phényl.

Synthèse

a) Préparation de l'amino-2 ((chloro-3 phényl)-4 pipérazinylméthyl)-5 oxazoline-2 ou COR3240 produit de structure II avec $Ar_1$ = chloro-3 phényl. Ce produit est préparé selon la méthode décrite dans l'exemple 6a en utilisant la (chloro-3 phényl) pipérazine comme produit de départ. Le COR3240 est purifié par recristallisation dans le trichloroéthylène, rendement 28%, masse moléculaire 294,5.

Caractéristiques physicochimiques du COR3240

Point de fusion 198 ° C
Spectre de RMN dans le DMSO $D_6$
2,3-4,0 ppm, 12 protons, massif compexe, 6 groupes $N-CH_2$; 4,3-4,9 ppm, 1 proton, multiplet, 1 CHO du cycle de l'oxazoline; 5,8 ppm, 2 protons échangeables avec $D_2O$, pic large, $NH_2$; 6,6-7,4 ppm, 4 protons, massif complexe, protons aromatiques.

b) Préparation de la phényl carbamoyl-3 imino-2 ((chloro-3 phényl)-4 pipérazinylméthyl)-5 oxazolidine. Ce produit est préparé selon la méthode décrite dans l'exemple 1 en utilisant le COR3240 et l'isocyanate de phényle comme produit de départ. Le produit final est purifié par lavage dans l'éthanol absolu, rendement 82%, masse moléculaire 413,5.

Caractéristiques physicochimiques du COR3279

Point de fusion 155 ° C
Spectre de RMN dans le $CDCl_3$.
2,5-3,5 ppm, 10 protons, massif complexe, 5 groupes $N-CH_2$; 3,6-4,5 ppm, 2 protons, massif complexe, 1 groupe $N-CH_2$ du cycle de l'oxazolidine; 4,5-4,9 ppm, 1 proton, multiplet, 1 CHO du cycle de l'oxazolidine; 6,0 ppm, 1 proton échangeable avec $D_2O$, pic large, NH du groupe imino; 6,6-7,8 ppm, 9 protons, massif complexe, protons aromatiques; 11,6 ppm, 1 proton échangeable avec $D_2O$, pic large, NH du groupe carbamoyl.

EXEMPLE 8

Chlorhydrate de la phényl carbamoyl-3 ((chloro-3 phényl)-4 pipérazinylméthyl)-5 oxazolidinone-2 ou COR3277 produit de structure I avec $R_1$ = O, $R_2$ = O, $Ar_1$ = chloro-3 phényl, $Ar_2$ = phényl.

Synthèse

On procéde de la même façon que dans l'exemple 2, en utilisant le COR3279 préparé dans l'exemple 7 comme produit de départ, on obtient après recristallisation dans l'éthanol absolu le COR3277 avec un rendement de 33%, masse moléculaire 451.

Caractéristiques physicochimiques du COR3277

Point de fusion 170 ° C
Spectre de RMN dans le DMSO $D_6$
3,0-4,5 ppm, 12 protons, massif complexe, 6 groupes $N-CH_2$; 5,2-5,7 ppm, 1 proton, multiplet, 1 CHO du cycle de l'oxazolidinone; 6,7-7,7 ppm, 9 protons, massif complexe, protons aromatiques; 9,8 ppm, 1 proton échangeable avec $D_2O$, pic large, NH du groupe carbamoyl; 12,1 ppm, 1 proton échangeable avec $D_2O$, dôme, 1 $NH^+$.

EXEMPLE 9

Phényl carbamoyl-3 imino-2 ((trifluorométhyl-3 phényl)-4 pipérazinylméthyl)-5 oxazolidine ou COR3266 produit de structure I avec $R_1$ = NH, $R_2$ = O, $Ar_1$ = trifluorométhyl-3 phényl, $Ar_2$ = phényl.

Synthèse

a) Préparation de l'amino-2 ((trifluorométhyl-3 phényl)-4 pipérazinylméthyl)-5 oxazoline-2 ou COR3235 produit de structure II avec $Ar_1$ = trifluorométhyl-3 phényl.

le COR3235 est préparé selon la méthode décrite dans l'exemple 6a en utilisant la trifluorométhyl-3 phényl pipérazine comme produit de départ. Le COR3235 est purifié par recristallisation dans le trichloroéthylène, rendement 22%, masse moléculaire 328.

Caractéristiques physicochimiques du COR3235

Point de fusion 163 °C
Spectre de RMN dans le DMSO $D_6$
2,3-4,0 ppm, 12 protons, massif complexe, 6 groupes N-$CH_2$; 4,4-4,9 ppm, 1 proton, multiplet, 1 CHO du cycle de l'oxazoline; 5,9 ppm, 2 protons échangeables avec $D_2O$, $NH_2$; 6,9-7,7 ppm, 4 protons, massif complexe, protons aromatiques.

b) Préparation de la phényl carbamoyl-3 imino-2 ((trifluorométhyl-3 phényl)-4 pipérazinylméthyl)-5 oxazolidine. Ce produit est préparé selon la méthode décrite dans l'exemple 1 en utilisant le COR3235 et l'isocyanate de phényle comme produits de départ. Le produit final est purifié par recristallisation dans l'oxyde de diisopropyle, rendement 44%, masse moléculaire 447.

Caractéristiques physicochimiques du COR3266

Point de fusion 150 °C
Spectre de RMN dans le $CDCl_3$
2,5-3,5 ppm, 10 protons, massif complexe, 5 groupes N-$CH_2$; 3,6-4,5 ppm, 2 protons, massif complexe, 1 groupe N-$CH_2$ du cycle de l'oxazolidine; 4,5-5,0 ppm, 1 proton, multiplet, 1 CHO du cycle de l'oxazolidine; 6 ppm, 1 proton échangeable avec $D_2O$, pic large, NH du groupe imino; 6,9-7,7 ppm, 9 protons, massif complexe, protons aromatiques; 11,6 ppm, 1 proton échangeable avec $D_2O$, pic large, NH du groupe carbamoyl.

EXEMPLE 10

Phényl carbamoyl-3 ((trifluorométhyl-3 phényl)-4 pipérazinylméthyl)-5 oxazolidinone-2 ou COR3276 produit de structure I avec $R_1$ = O $R_2$ = O, $Ar_1$ = trifluorométhyl-3 phényl, $Ar_1$ = phényl.

Synthèse

On procéde de la même façon que dans l'exemple 2, en utilisant le COR3266 préparé dans l'exemple 9 comme produit de départ. On obtient après recristallisation dans l'éthanol absolu le COR3276 avec un rendement de 49%, masse moléculaire 448.

Caractéristiques physicochimiques du COR3276

Point de fusion 173 °C
Spectre de RMN dans le DMSO $D_6$.
2,4-3,5 ppm, 10 protons, massif complexe, 5 groupes N-$CH_2$; 3,5-4,4 ppm, 2 protons, massif complexe, 1 groupe N-$CH_2$ du cycle de l'oxazolidinone; 4,7-5,2 ppm, 1 proton, multiplet, 1 CHO du cycle de l'oxazolidinone; 6,9-7,7 ppm, 9 protons, massif complexe, protons aromatiques; 9,9 ppm, 1 proton échangeable avec $D_2O$, pic large, NH du groupe carbamoyl.

EXEMPLE 11

Phényl carbamoyl-3 imino-2 ((fluoro-4 phényl)-4 pipérazinylméthyl)-5 oxazolidine ou COR3278 produit de structure I avec $R_1$ = NH; $R_2$ = O, $Ar_1$ = fluoro-4 phényl, $Ar_2$ = phényl.

Synthèse

a) Préparation de l'amino-2 ((fluoro-4 phényl)-4 pipérazinylméthyl)-5 oxazoline ou COR3242 produit de stucture II avec $Ar_1$ = fluoro-4 phényl.
Le COR3242 est préparé selon la méthode décrite dans l'exemple 6a en utilisant la fluoro-4 phényl pipérazine comme produit de départ. Le COR3242 est purifié par recristallisation dans l'heptane,rendement 28%, masse moléculaire 278.

Caractéristiques physicochimiques du COR3242

Point de fusion 170 ° C
Spectre de RMN dans le DMSO $D_6$
2,2-3,9 ppm, 12 protons, massif complexe, 6 groupes $N-CH_2$; 4,3-4,9 ppm, 1 proton, multiplet, 1 CHO du cycle de l'oxazoline; 5,9 ppm, 2 protons échangeables avec $D_2O$, $NH_2$; 6,7-7,3 ppm, 4 protons, massif complexe, protons aromatiques.

b) Préparation de la phényl carbamoyl-3 imino-2 ((fluoro-4 phényl)-4 pipérazinylméthyl)-5 oxazolidine.
Ce produit est préparé selon la méthode décrite dans l'exemple 1 en utilisant le COR3242 et l'isocyanate de phényle comme produit de départ. le produit final est purifié par lavage dans de l'éthanol absolu, rendement 65%, masse moléculaire 397.

Caractéristiques physicochimiques du COR3278

Point de fusion 169 ° C
Spectre de RMN dans le $CDCl_3$.
2,5-3,3 ppm, 10 protons, massif complexe, 5 groupes $N-CH_2$: 3,6-4,5 ppm, 2 protons, massif complexe, 1 groupe $N-CH_2$ du cycle de l'oxazolidine; 4,5-5,0 ppm, 1 proton, multiplet, 1 CHO du cycle de l'oxazolidine; 6,0 ppm, 1 proton échangeable avec $D_2O$, pic large, NH du groupe imino; 6,7-7,7 ppm , 9 protons, massif complexe, protons aromatiques; 11,6 ppm, 1 proton échangeable avec $D_2O$ pic large, NH du groupe carbamoyl.

EXEMPLE 12

Chlorhydrate de la phényl carbamoyl-3 ((fluoro-4 phényl)-4 pipérazinylméthyl)-5 oxazolidinone-2 ou COR3265 produit de structure I avec $R_1$ = O, $R_2$ = O, $Ar_1$ = fluoro-4 phényl, $Ar_2$ = phényl.

Synthèse

On procéde de la même façon que dans l'exemple 2 en utilisant le COR3278 préparé dans l'exemple 11 comme produit de départ. On obtient après recristallisation dans l'éthanol absolu le COR3265 avec un rendement de 95%, masse moléculaire 434,5.

Caractéristiques physicochimiques du COR3265

Point de fusion 210 ° C
Spectre de RMN dans le DMSO $D_6$
2,9-4,5 ppm, 12 protons, massif complexe, 6 groupes $N-CH_2$; 5,2-5,8 ppm, 1 proton, multiplet, 1 CHO du cycle de l'oxazolidinone; 6,8-7,7 ppm, 9 protons, massif complexe, protons aromatiques; 9,8 ppm 1 proton échangeable avec $D_2O$ pic large, NH du groupe carbamoyl; 12,1 ppm, 1 proton échangeable avec $D_2O$ pic étalé $NH^+$.

EXEMPLE 13

Phényl carbamoyl-3 imino-2 ((diméthyl-2,3 phényl)-4 pipérazinylméthyl)-5 oxazolidine ou COR4417 produit de structure I avec $R_1$ = NH, $R_2$ = O, $Ar_1$ = diméthyl-2,3 phényl, $Ar_2$ = phényl.

Synthèse

a) Préparation de l'amino-2 ((dimethyl-2,3 phényl)-4 pipérazinylméthyl)-5 oxazoline-2 ou COR3291 produit de structure II avec $Ar_1$ = diméthyl-2,3 phényl.
Ce produit est préparé selon la méthode décrite dans l'exemple 6a en utilisant la (diméthyl-2,3 phényl) pipérazine comme produit de départ. Le produit final est purifié par recristallisation dans l'heptane, rendement 23% masse moleculaire 288.

Caractéristiques physicochimiques du COR3291

Point de fusion 149 ° C
Spectre de RMN dans le $CDCl_3$
2,2 ppm, 3 protons, singulet, 1 groupe $CH_3$; 2,3 ppm, 3 protons, singulet, 1 groupe $CH_3$; 2,5-3,2 ppm, 10 protons, massif complexe, 5 groupes N-$CH_2$; 3,2-4,1 ppm, 2 protons, massif complexe, 1 groupe N-$CH_2$ du cycle de l'oxazoline; 4,5-5,0 ppm, 1 proton, multiplet, 1 CHO du cycle de l'oxazoline; 5,1 ppm, 2 protons, pic large échangeable avec $D_2O$, $NH_2$; 6,7-7,3 ppm, 3 protons, massif complexe, protons aromatiques.

b) préparation de la phényl carbamoyl-3 imino-2 ((diméthyl-2,3 phényl)-4 pipérazinylméthyl)-5 oxazolidine. Ce produit est préparé selon la méthode décrite dans l'exemple 1 en utilisant le COR3291 et l'isocyanate de phényle comme produits de départ. Le produit final est purifié par lavage dans l'éthanol absolu, rendement 64%, masse moléculaire 407.

Caractéristiques physicochimiques du COR4417

Point de fusion 155 ° C
Spectre de RMNdans le $CDCl_3$
2,2 ppm, 3 protons, singulet, 1 groupe $CH_3$; 2,3 ppm, 3 protons, singulet, 1 groupe $CH_3$; 2,6-3,0 ppm, 10 protons, massif complexe, 5 groupes N-$CH_2$; 3,6-4,4 ppm, 2 protons, massif complexe, 1 groupe N-$CH_2$ du cycle de l'oxazolidine; 4,5-4,9 ppm, 1 proton, multiplet, 1 CHO du cycle de l'oxazolidine; 6,0 ppm, 1 proton échangeable avec $D_2O$, pic large, NH du groupe imino; 6,8-7,6 ppm, 8 protons , massif complexe, protons aromatiques; 11,6 ppm, 1 proton échangeable avec $D_2O$, pic large, NH du groupe carbamoyl.

EXEMPLE 14

Phényl carbamoyl-3 imino-2 ((pyrimidinyl-2)-4 pipérazinylméthyl)-5 oxazolidine ou COR4421 produit de structure I avec $R_1$ = NH, $R_2$ = O, $Ar_1$ = pyrimidyl-2, $Ar_2$ = phényl.

Synthèse

a) Préparation de l' amino-2 ((pyrimidinyl-2)-4 pipérazinylméthyl)-5 oxazoline ou COR3286 produit de structure III. Le COR3286 est synthétisé selon la méthode décrite dans l'exemple 1 du brevet FR 83 08477 à partir de la pyrimidinyl-2 pipérazine on obtient le COR3286 avec un rendement de 49%.

Caractéristiques physicochimiques du COR3286

Point de fusion 182 ° C
Spectre de RMN dans le DMSO $D_6$
2,2-4 ppm, 12 protons, massif complexe, 6 groupe N-$CH_2$; 4,3-4,9 ppm, 1 proton, multiplet, 1 CHO du cycle de l'oxazoline; 5,5 ppm, 2 protons échangeables avec $D_2O$, pic large, $NH_2$; 6,6 ppm, 1 proton, triplet, 1 CH en position 4 de la pyrimidine; 8,3 ppm, 2 protons, doublet, 2 CH en position 3, 5 de la pyrimidine.

b) Préparation de la phényl carbamoyl -3 imino-2 ((pyrimidinyl-2)-4 pipérazinylméthyl)-5 oxazoline ou COR4421.

Dans un réacteur muni d' un réfrigérant, d'une ampoule à bromé, d'un agitateur et d'un thermomètre on introduit 26,2 g (0,1 mole) du COR3286 préalablement dissous dans 150 ml de benzène anhydre. La solution est refroidie à 277 ° K puis on ajoute goutte à goutte 11,9 g (0,1 mole) d'isocyanate de phényle en contrôlant la température celle ci ne devant pas dépasser 293 ° K. Le mélange est laissé sous agitation pendant 25 minutes. Le solide qui a précipité est receuilli par filtration et est recristallisé dans l'oxyde de diisopropyle. On receuille 19 grammes de COR4421, rendement 51% masse moléculaire 381,44.

Caractéristiques physicochimiques du COR4421

Point de fusion 160 ° C
Spectre de RMN dans le $CDCl_3$
2,3-4,5 ppm, 12 protons, massif complexe, 6 N-$CH_2$; 4,5-4,9 ppm, 1 proton, multiplet, 1 CHO du cycle de l'oxazolidine; 6,1 ppm, 1 proton échangeable avec $D_2O$, pic large, NH du groupe imino; 6,5 ppm, 1 proton, triplet, 1 CH en position 4 de la pyrimidine; 7,0-7,7 ppm, 5 protons, massif complexe, protons aromatiques; 8,3 ppm, 2 protons, doublet, 2 CH en position 3, 5 de la pyrimidine; 11,7 ppm, 1 proton échangeable avec

$D_2O$, pic large, NH du groupe carbamoyle.

TOXICITE

Administrés chez la souris par voie orale à la dose de 300mg/kg et par voie intrapéritonéale à la dose de 200mg/kg les COR3262, COR3263, COR3265, COR3266, COR3267, COR3276, COR3277, COR3278, COR3279, COR3296 n'entrainent aucune mortalité.

PHARMACOLOGIE

Administré chez le rat génétiquement hypertendu à la dose de 60mg/kg par voie orale le COR3267 entraine une diminution de la pression artérielle de 8, 12 et 14 %, ces variations étant respectivement mesurées 2, 4 et 6 heures après l'administration.

In vitro une activité antifongique a été mise en évidence vis à vis de candida albicans pour le COR3296. L'activité observée par des techniques de dilution standard est significative à la concentration de 20 microgramme par ml.

Compte tenu de leurs activités pharmacotoxicologiques les produits faisant l'objet de la présente invention sont utiles, seuls ou associés à d'autres principes actifs, par exemple dans le traitement de l'hypertension artérielle ou dans le traitement des candidoses.

Les doses et les schémas thérapeutiques seront fonction du sujet et de l'affection à traiter. Les produits pouront être administrés par voie orale (par exemple sous forme de gélules, comprimés, gouttes buvables), par voie injectable (soluté injectable pour la voie intramusculaire ou la voie intraveineuse; soluté pour la perfusion intraveineuse), par voie rectale (suppositoires) par voie locale (collyres, crèmes, pomades, gels). Suivant les indications, la dose quotidienne variera de 1 à 100 mg de une à quatre fois par jour.

**Revendications**

**Revendications pour les Etats contractants suivants : AT, BE, CH, DE, GB, IT, LI, LU, NL**

**1.** Produits de formule générale :

(I)

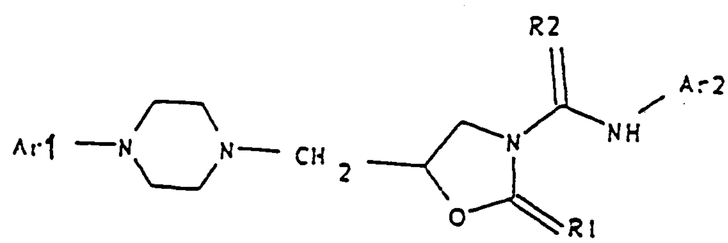

dans laquelle $R_1$ est un oxygène ou un groupe NH , $R_2$ est un oxygène ou un soufre, $Ar_1$ est un groupe phényl substitué ou non par un ou plusieurs groupes choisis parmi des groupes alkyl de $C_1$ à $C_4$, halogène, chlore, brome, fluor, un groupe trifluorométhyl ou un groupe méthoxy, $Ar_1$ est aussi un groupe pyridyl ou pyrimidyl; le groupe $Ar_2$ est un groupe phényl substitué ou non par un ou plusieurs groupes choisis parmi des groupes alkyl de $C_1$ à $C_4$ , chlore, brome, fluor, iode.

**2.** Méthode de préparation des produits selon la revendication 1, caractérisée en ce que l'on fait reagir dans un milieu anhydre à la température de 233°K à 293°K, l'isocyanate d'aryle ou l'isothiocyanate d'aryle de formule (III) $Ar_2\text{-}N=C=R_2$ avec un dérivé de formule générale:

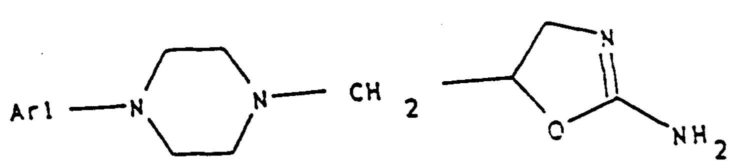

(II)

dans lesquels $R_2$, $Ar_1$ et $Ar_2$ ont les significations données à la revendication 1.

**3.** Méthode selon la revendication 2 de préparation des produits selon la revendication 1 , caractérisée en ce que le groupe $Ar_1$ est un groupe pyridyl.

**4.** Méthode selon la revendication 2 de préparation des produits selon la revendication 1, caractérisée en ce que le groupe $Ar_1$ est un groupe pyrimidyl.

**5.** Méthode selon la revendication 2 de preparation des produits selon la revendication 1, caractérisée en ce que l'on hydrolyse par l'acide chlorhydrique dans un solvant à l'ébullition des produits préparés selon la revendication 2.

**6.** Nouveau médicament caractérisé en ce que le principe actif est constitué par au moins un produit selon la revendication 1.

**7.** Nouveau médicament selon la revendication 6 utile pour le traitement de l'hypertension.

**8.** Nouveau médicament selon la revendication 6 utile pour le traitement des candidoses.

**9.** Composition pharmaceutique ou vétérinaire caractérisée en ce qu'elle contient à titre de principe actif au moins un produit selon la revendication 1 en association avec un véhicule pharmaceutique ou un excipient approprié.

**Revendications pour l'Etat contractant suivant : ES**

**1.** Méthode de préparation des produits de formule générale :

(I)

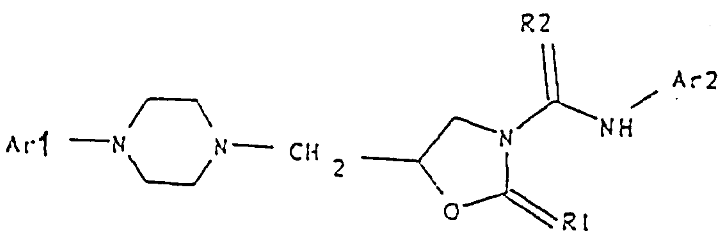

dans laquelle $R_1$ est un oxygene ou un groupe NH, $R_2$ est un oxygène ou un soufre, $Ar_1$ est un groupe phényl substitué ou non par un ou plusieurs groupes choisis parmi des groupes alkyl de C1 à C4, halogène, chlore, brome, fluor, un groupe trifluorométhyl ou un groupe méthoxy, $Ar_1$ est aussi un groupe pyridyl ou pyrimidyl; le groupe $Ar_2$ est un groupe phényl substitué ou non par un ou plusieurs groupes choisis parmi des groupes alkyl de C1 à C4, chlore, brome, fluor, iode, caractérisée en ce que l'on fait réagir dans un milieu anhydre à la température de 233 °K à 293 °K, l'isocyanate d'aryle ou l'isothiocyanate d'aryle de formule (III) $Ar_2-N=C=R_2$ avec un dérivé de formule générale :

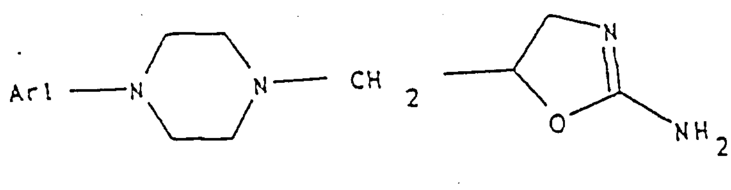

(II)

**2.** Méthode selon la revendication 1 caractérisée en ce que le groupe $Ar_1$ est un groupe pyridyl.

**3.** Méthode selon la revendication 1 caractérisée en ce que le groupe $Ar_1$ est un groupe pyrimidyl.

**4.** Méthode selon la revendication 1 caractérisée en ce que l'on hydrolyse par l'acide chlorhydrique dans un solvant à l'ébullition des produits préparés selon la revendication 1.

**5.** Méthode de préparation d'une composition pharmaceutique ou d'un médicament, caractérisée en ce que l'on associe un produit selon la revendication 1 avec un véhicule pharmaceutique ou un excipient approprié.

**6.** Méthode selon la revendication 5, caractérisée en ce que la composition ou le médicament sont destinés au traitement de l'hypertension ou des candidoses.

**Claims**
**Claims for the following Contracting States : AT, BE, CH, DE, GB, IT, LI, LU, NL**

**1.** Products of general formula :

(I)

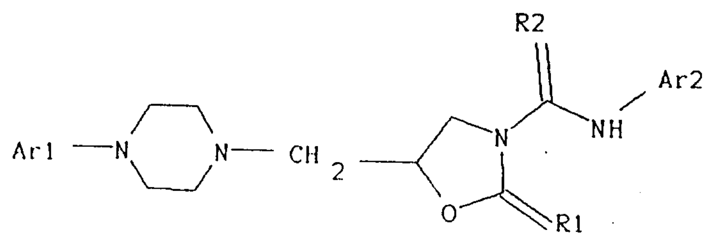

in which $R_1$ is oxygen or an NH group, $R_2$ is oxygen or sulfur, $Ar_1$ is a phenyl group substituted or not by one or more groups selected from $C_1$ to $C_4$ alkyl groups, halogen, chlorine, bromine, fluorine, a trifluoromethyl group or a methoxy group, $Ar_1$ is also a pyridyl or pyrimidyl group; the $Ar_2$ group is a phenyl group substituted or not by one or more groups selected from $C_1$ to $C_4$ alkyl groups, chlorine, bromine, fluorine, iodine.

**2.** Method of preparation of products according to claim 1, characterized in that the aryl isocyanate or the aryl isothiocyanate of formula (III) $Ar_2$-N=C=$R_2$ is caused to react in anhydrous medium at a temperature of 233°K to 293°K with a derivative of general formula :

(II)

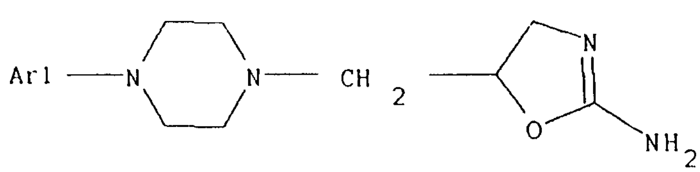

in which $R_2$, $Ar_1$ and $Ar_2$ have the meanings given in claim 1.

**3.** Method according to claim 2 of preparation of products according to claim 1, characterized in that the $Ar_1$ group is a pyridyl group.

**4.** Method according to claim 2 of preparation of products according to claim 1, characterized in that the $Ar_1$ group is a pyrimidyl group.

**5.** Method according to claim 2 of preparation of products according to claim 1, characterized in that the products prepared according to claim 2 are hydrolyzed by hydrochloric acid in a boiling solvent.

**6.** New drug characterized in that the active ingredient is composed of at least one product according to claim 1.

**7.** New drug according to claim 6 useful for the treatment of hypertension.

**8.** New drug according to claim 6 useful for the treatment of candidiasis.

**9.** Pharmaceutical or veterinary composition characterized in that it contains as active ingredient at least one product according to claim 1 in combination with a pharmaceutical vehicle or an appropriate excipient.

**Claims for the following Contracting State : ES**

**1.** Method of preparation of products of general formula :

(I)

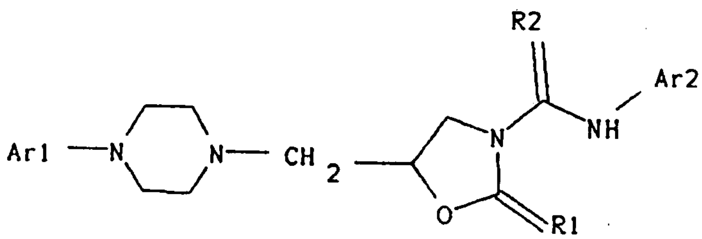

in which $R_1$ is oxygen or an NH group, $R_2$ is oxygen or sulfur, $Ar_1$ is a phenyl group substituted or not by one or more groups selected from $C_1$ to $C_4$ alkyl groups, halogen, chlorine, bromine, fluorine, a trifluoromethyl group or a methoxy group, $Ar_1$ is also a pyridyl or pyrimidyl group; the $Ar_2$ group is a phenyl group substituted or not by one or more groups selected from $C_1$ to $C_4$ alkyl groups, bromine, fluorine, iodine, characterized in that the aryl isocyanate or the aryl isothiocyanate of formula (III) $Ar_2$-$N=C=R_2$ is caused to react in anhydrous medium at a temperature of 233°K to 293°K with a derivative of general formula :

(II)

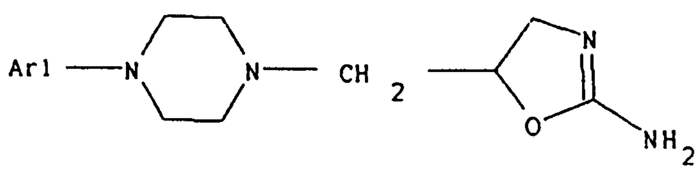

**2.** Method according to claim 1 characterized in that the $Ar_1$ group is a pyridyl group.

**3.** Method according to claim 1 characterized in that the $Ar_1$ group is a pyrimidyl group.

**4.** Method according to claim 1 characterized in that the products prepared according to claim 1 are hydrolyzed by hydrochloric acid in a boiling solvent.

**5.** Method of preparation of a pharmaceutical composition or a drug, characterized in that it contains a product according to claim 1 in combination with a pharmaceutical vehicle or an appropriate excipient.

**6.** Method according to claim 5, characterized in that the composition or the drug are intended for the treatment of hypertension or candidiasis.

**Patentansprüche**
**Patentansprüche für folgende Vertragsstaaten : AT, BE, CH, DE, GB, IT, LI, LU, NL**

1. Produkte der allgemeinen Formel:

( I )

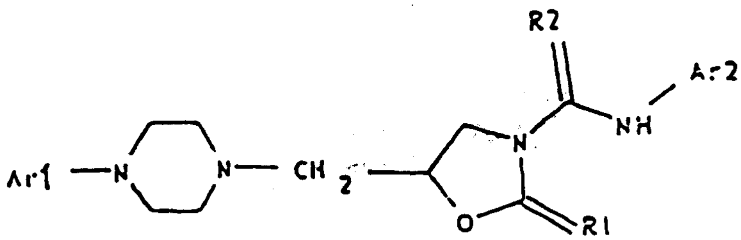

worin $R_1$ ein Sauerstoff oder ein NH-Rest ist, $R_2$ ein Sauerstoff oder ein Schwefel ist, $Ar_1$ ein Phenylrest ist, der gegebenenfalls substituiert ist durch einen oder mehrere Reste, ausgewählt aus den Resten Alkyl mit $C_1$ bis $C_4$, Halogen, Chlor, Brom, Fluor, einem Trifluormethyl- oder einem Methoxyrest, $Ar_1$ auch ein Pyridyl- oder Pyrimidylrest ist; der Rest $Ar_2$ ein gegebenenfalls durch einen oder mehrere Reste, ausgewählt aus den Resten Alkyl mit $C_1$ bis $C_4$, Chlor, Brom, Fluor,Iod, substituierter Phenylrest ist.

2. Verfahren zur Herstellung von Produkten gemäß Anspruch 1, dadurch gekennzeichnet, daß man in einem wasserfreien Milieu bei einer Temperatur von 233 K bis 293 K Arylisocyanat oder Arylisothiocyanat der Formel (III) $Ar_2\text{-}N=C=R_2$ mit einem Derivat der allgemeinen Formel:

(II)

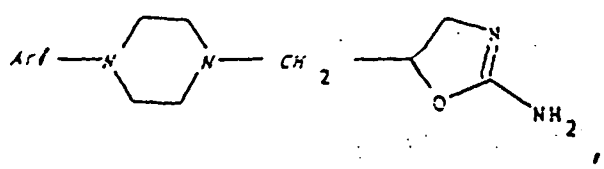

worin $R_2$, $Ar_1$ und $Ar_2$ die im Anspruch 1 angegebenen Bedeutungen haben, umsetzt.

3. Verfahren gamäß Anspruch 2 zur Herstellung von Produkten nach Anspruch 1, dadurch gekennzeichnet, daß der Rest $Ar_1$ ein Pyridyl-Rest ist.

4. Verfahren gemäß Anspruch 2 zur Herstellung von Produkten nach Anspruch 1, dadurch gekennzeichnet, daß der Rest $Ar_1$ ein Pyrimidyl-Rest ist.

5. Verfahren gemäß Anspruch 2 zur Herstellung von Produkten nach Anspruch 1, dadurch gekennzeichnet, daß man gemäß Anspruch 2 hergestellte Produkte mittels Chlorwasserstoff-Säure in einem siedenden Lösungsmittel hydrolysiert.

6. Neues Medikament, dadurch gekennzeichnet, daß der Wirkstoff zumindestens ein Produkt gemäß Anspruch 1 umfaßt.

7. Neues Medikament gemäß Anspruch 6, das für die Behandlung von Bluthochdruck geeignet ist.

8. Neues Medikament gemäß Anspruch 6, das für die Behandlung von Candidiosen geeignet ist.

9. Pharmazeutische oder veterinärische Zusammensetzung, dadurch gekennzeichnet, daß sie als Wirkstoff wenigstens ein Produkt gemäß Anspruch 1 in Verbindung mit einem pharmazeutischen Vehikel oder einem geeigneten Hilfsstoff enthält.

**Patentansprüche für folgenden Vertragsstaat : ES**

1. Verfahren zur Herstellung von Produkten der allgemeinen Formel:

(I)

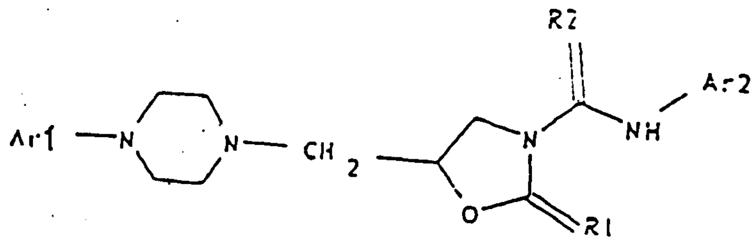

worin $R_1$ ein Sauerstoff oder ein NH-Rest ist, $R_2$ ein Sauerstoff oder ein Schwefel ist, $Ar_1$ ein Phenylrest ist, der gegebenenfalls substituiert ist durch einen oder mehrere Reste, ausgewählt aus den Resten Alkyl mit $C_1$ bis $C_4$, Halogen, Chlor, Brom, Fluor, einem Trifluormethyl- oder einem Methoxyrest, $Ar_1$ auch ein Pyridyl- oder Pyrimidylrest ist; der Rest $Ar_2$ ein gegebenenfalls durch einen oder mehrere Reste, ausgewählt aus den Resten Alkyl mit $C_1$ bis $C_4$, Chlor, Brom, Fluor, Iod, substituierter Phenylrest ist, dadurch gekennzeichnet, daß man in einem wasserfreien Milieu bei einer Temperatur von 233 K bis 293 K Arylisocyanat oder Arylisothiocyanat der Formel (III) $Ar_2\text{-}N=C=R_2$ mit einem Derivat der allgemeinen Formel:

(II)

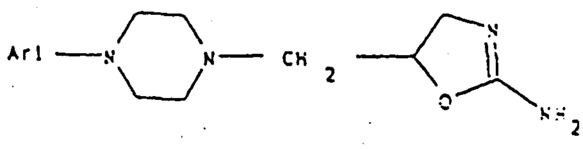

umsetzt.

2. Verfahren gemäß Anspruch 1, dadurch gekennzeichnet, daß der Rest $Ar_1$ ein Pyridyl-Rest ist.

3. Verfahren gemäß Anspruch 1, dadurch gekennzeichnet, daß der Rest $Ar_1$ ein Pyrimydyl-Rest ist.

4. Verfahren gemäß Anspruch 1, dadurch gekennzeichnet, daß man mittels Chlorwasserstoff-Säure in einem siedenden Lösungsmittel nach Anspruch 1 hergestellte Produkte hydrolysiert.

5. Verfahren zur Herstellung einer pharmazeutischen Zusammensetzung oder eines Medikaments, dadurch gekennzeichnet, daß man ein Produkt gemäß Anspruch 1 mit einem pharmazeutischen Vehikel oder einem geeigneten Hilfsstoff verbindet.

6. Verfahren gemäß Anspruch 5, dadurch gekennzeichnet, daß die Zusammensetzung oder das Medikament zur Behandlung von Bluthochdruck oder Candidiosen bestimmt ist.